# EUROPEAN PATENT APPLICATION

(11) **EP 4 420 634 A1**
(43) Date of publication of application: **28.08.2024**
(21) Application number: 22897520.7
(22) Date of filing: 26.10.2022
(51) Int. Cl.: A61F 2/07

(54) **COVERED STENT AND COVERED STENT SYSTEM**

(30) Priority: 26.11.2021 CN 202111424608
(71) Applicant: Shanghai MicroPort Endovascular MedTech (Group) Co., Ltd., Shanghai 201318 (CN)
(72) Inventor: ZHANG, Bowei, Shanghai 201318 (CN); TU, Chunlin, Shanghai 201318 (CN); ZHOU, Xingyu, Shanghai 201318 (CN); ZHU, Qing, Shanghai 201318 (CN)
(74) Representative: Arnold & Siedsma
(86) International application number: PCT/CN2022/127687
(87) International publication number: WO 2023/093437

(57) **Abstract**

A covered stent (10) and a covered stent system. The covered stent (10) comprises a metal stent (100) and a covering film (200) covering the metal stent (100); the covering film (200) comprises a proximal end (201) and a distal end (202); the covering film (200) comprises a hole area (210) close to the distal end (202); the hole area (210) is provided with a plurality of through holes (240); blood can flow from the inside of the metal stent (100) to the outside via the through holes (240).

## Description

### CROSS-REFERENCE TO RELATED APPLICATIONS

This application claims priority of Chinese Patent Application No. 2021114246083, filed on November 26, 2021, entitled "COVERED STENT AND COVERED STENT SYSTEM", the entire content of which is incorporated herein in its entirety.

### TECHNICAL FIELD

The present disclosure relates to medical devices, in particular to a stent graft and a stent graft system.

### BACKGROUND

Descending aortic dissecting aneurysm is a vascular disease, which is generally caused by hypertension, connective tissue genetic diseases, some congenital cardiovascular diseases (such as coarctation of the descending aorta, bivalve of the descending aorta), or pregnancy, severe trauma, heavy physical labor and some drugs. Treatment methods of the descending aortic dissecting aneurysm includes drug therapy, open surgery and interventional surgery. Currently, a preferred treatment for Stanford type B (equivalent to DeBakey type III) is percutaneous stent graft placement, with surgical treatment if necessary.

The therapeutic principle of stent grafts is to prevent a rupture of a dissecting aneurysm by replacing or repairing a part of a blood vessel, such as an arterial blood vessel. The stent graft includes a tubular membrane and a plurality of stent segments. However, when the stent graft is placed in the descending aorta and extended to a distal intercostal artery, it is very likely that the distal intercostal artery located in the descending aorta will be occluded, resulting in spinal cord ischemia (SCI). The spinal cord ischemia complicated by paraplegia has been recognized as a terrible complication after descending thoracic aortic surgery.

Currently, in order to solve the problem of spinal cord ischemia, a metal stent of the stent graft is generally protruded from the distal end of the stent graft to form a bare metal stent. The bare metal stent is placed at a distant intercostal artery to play a supporting role and does not affect the blood flow supply of the branch vessel. The bare metal stent is either made of overlapping and weaving metal, or the plurality of stent segments bundled by polymer wires. For the bare metal stent made of overlapping and weaving metal, a long-term fatigue performance is poor and easy to break, which makes the metal stent fail and endangers the blood vessel. For the bare metal stent made of the stent segments, a connection strength between stent segments cannot be guaranteed, the polymer wire is easily broken due to external force, and the polymer wire needs a strong fixing ability to fix the stent segments to ensure that there is no displacement of the metal stent.

### SUMMARY

According to some embodiments, a stent graft and a stent graft system are provided.

A stent graft includes a metal stent, and a membrane wrapping the metal stent and including a proximal end and a distal end. The membrane includes a hole area adjacent to the distal end, the hole area is provided with a plurality of through holes configured to allow blood to flow from an inside of the metal stent to an outside thereof.

In one of the embodiments, the metal stent includes a plurality of stent segments spaced apart in an axial direction thereof, each stent segment has a plurality of edges and a plurality of vertices, and the hole area includes a first area and a second area, the first area is adjacent to the second area, the second area covers one of the vertices, an area of the through hole in the first area is greater than an area of the through hole in the second area.

In one of the embodiments, the through hole in the first area is a first circular hole, a diameter of the first circular hole is in a range of 1mm to 6mm, a distance between centers of adjacent two first circular holes is in a range of 4mm to 6mm, the centers of adjacent three first circular holes form an equilateral triangle.

In one of the embodiments, the second area includes an area with the vertex as a center and a radius of 5mm to 7mm, the through hole in the second area is a second circular hole, a diameter of the second circular hole is 40% to 70% less than the diameter of the first circular hole.

In one of the embodiments, a distance between any point on an edge of the second circular hole and the vertex is greater than or equal to 3cm.

In one of the embodiments, the through hole in the first area is a first diamond-shaped hole, an area of the first diamond-shaped hole is in a range of 0.78mm² to 7mm², an edge distance between adjacent two first through holes is in a range of 1mm to 3mm, the first diamond-shaped holes are arranged in a matrix.

In one of the embodiments, the second area includes an area with the vertex as a center and a radius of 5mm to 7mm, the through hole in the second area is a second diamond-shaped hole, an area of the second diamond-shaped hole is 40% to 70% less than the area of the first diamond-shaped hole.

In one of the embodiments, a distance between any point on an edge of the second diamond-shaped hole and the vertex is greater than or equal to 3cm.

In one of the embodiments, the metal stent includes a plurality of stent segments spaced apart in an axial direction thereof, each stent segment has a plurality of edges and a plurality of vertices, the hole area is located between adjacent two edges of each stent segment, the through hole in the hole area is an amorphous hole, an area of the amorphous hole is in a range of 0.78mm² to 7mm², an edge distance between adjacent two amorphous holes is in a range of 1mm to 3mm.

In one of the embodiments, a distance between any point on an edge of the amorphous hole and the vertex is greater than or equal to 3cm.

In one of the embodiments, the areas of the amorphous holes gradually increase along a direction away from the vertex between the adjacent two edges.

In one of the embodiments, the stent graft further includes a first marker member provided on the membrane, the first marker member is located at a starting end of the hole area adjacent to the distal end and/or an ending end of hole area adjacent to the proximal end.

In one of the embodiments, the stent graft further includes a second marker member provided on the membrane, the second marker member is located on an edge of the membrane adjacent to the proximal end and/or the distal end.

A stent graft system includes a main stent graft and the above-mentioned stent graft, the main stent graft and the stent graft are sequentially arranged along a blood flow direction.

### BRIEF DESCRIPTION OF THE DRAWINGS

To illustrate the technical solutions according to the embodiments of the present invention or in the prior art more clearly, the accompanying drawings for describing the embodiments or the prior art are introduced briefly in the following. Apparently, the accompanying drawings in the following description are only some embodiments of the present invention, and persons of ordinary skill in the art can derive other drawings from the accompanying drawings without creative efforts.
FIG. 1 is a partial schematic view of a stent graft according to an embodiment of the present disclosure.
FIG. 2 is a schematic view of a stent graft according to an embodiment of the present disclosure.
FIG. 3 is a schematic view of the stent graft in FIG. 2 mounted in a descending aortic dissecting aneurysm.
FIG. 4 is a schematic view of the stent graft according to another embodiment of the present disclosure.
FIG. 5 is a schematic view of the stent graft in FIG. 4 mounted in a descending aortic dissecting aneurysm.
FIG. 6 is a schematic view of the stent graft in FIG. 4 mounted in a descending aortic dissecting aneurysm according to another embodiment of the present disclosure.
FIG. 7 is a partial enlarged view of the stent graft according to an embodiment of the present disclosure.
FIG. 8 is another partial enlarged view of the stent graft according to an embodiment of the present disclosure.
FIG. 9 is a partial enlarged view of the stent graft according to another embodiment of the present disclosure.
FIG. 10 is a partial enlarged view of the stent graft according to another embodiment of the present disclosure.

### DETAILED DESCRIPTION OF THE EMBODIMENTS

Embodiments of the invention are described more fully hereinafter with reference to the accompanying drawings. The various embodiments of the invention may, however, be embodied in many different forms and should not be construed as limited to the embodiments set forth herein. Rather, these embodiments are provided so that this disclosure will be thorough and complete, and will fully convey the scope of the invention to those skilled in the art. Elements that are identified using the same or similar reference characters refer to the same or similar elements.

In the description of the present disclosure, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential direction" are based on the azimuth or position relationship shown in the attached drawings, which is only for the convenience of describing the present disclosure and simplifying the description, rather than indicating or implying that the device or element must have a specific azimuth, be constructed and operated in a specific azimuth, so it cannot be understood as a limitation of the present disclosure.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include at least one of the features. In the description of the present disclosure, "multiple" means at least two, such as two, three, etc., unless otherwise expressly and specifically defined.

In the present invention, unless otherwise expressly specified and limited, the terms "install", "connect", "contact", "fix" and other terms should be understood in a broad sense, for example, they can be fixed connections, removable connections, or integrated. It can be mechanical connection or electrical connection. It can be directly connected or indirectly connected through an intermediate medium. It can be the connection within two elements or the interaction relationship between two elements, unless otherwise expressly limited. For those skilled in the art, the specific meaning of the above terms in the present disclosure can be understood according to the specific situation.

In the present invention, unless otherwise expressly specified and limited, the first feature "above" or "below" the second feature may be in direct contact with the first and second features, or the first and second features may be in indirect contact through an intermediate medium. Moreover, the first feature is "above" the second feature, but the first feature is directly above or diagonally above the second feature, or it only means that the horizontal height of the first feature is higher than the second feature. The first feature is "below" of the second feature, which can mean that the first feature is directly below or obliquely below the second feature, or simply that the horizontal height of the first feature is less than that of the second feature.

It should be noted that when an element is called "fixed to" or "disposed on" another element, it can be directly on another element or there can be a centered element. When an element is considered to be "connected" to another element, it can be directly connected to another element or there may be intermediate elements at the same time. The terms "vertical", "horizontal", "up", "down", "left", "right" and similar expressions used herein are for the purpose of illustration only and do not represent the only embodiment.

A descending aortic dissecting aneurysm refers to a condition in which blood in the lumen of the descending aorta enters a middle membrane of the descending aorta from an intimal tear of the descending aorta, causing the middle membrane to be separated and expanded along a long axis of the descending aorta to form a true and false two-lumen separation state of a descending aortic wall. To prevent rupture of the descending aortic dissecting aneurysm, a stent graft is placed in the true lumen of the descending aorta. However, when the stent graft is placed in the descending aorta and extends to a distant intercostal artery (such as the 8th to 11th intercostal arteries), the intercostal artery may be occluded by a conventional stent graft so that there is insufficient blood to pass through the intercostal arteries, resulting in spinal cord ischemia.

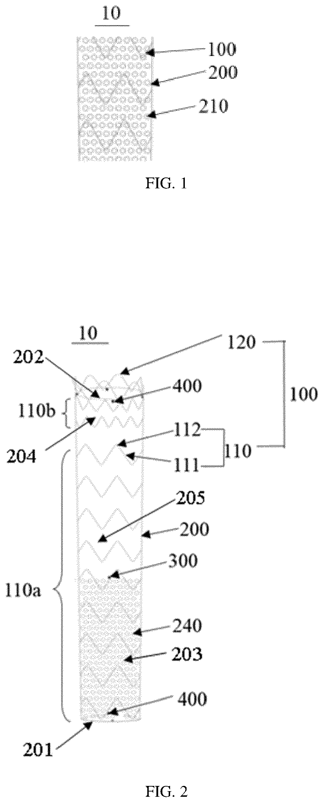

Accordingly, as shown in FIGS. 1 and 2, a stent graft 10 is provided according to an embodiment of the present disclosure, which includes a metal stent 100 and a membrane 200 wrapping the metal stent 100. The membrane 200 includes a proximal end 202 and a distal end 201. The distal end of the membrane 200 is defined as an end of the membrane 200 away from a heart, and the proximal end of the membrane 200 is defined as an end of the membrane 200 adjacent to the heart. The membrane 200 includes a hole area 210 adjacent to the distal end 201, and the hole area 210 is provided with a plurality of through holes 240. When the stent graft 10 is mounted in a descending aortic dissecting aneurysm, blood can flow from an inside of the stent graft 10 to an outside thereof through the through holes 240.

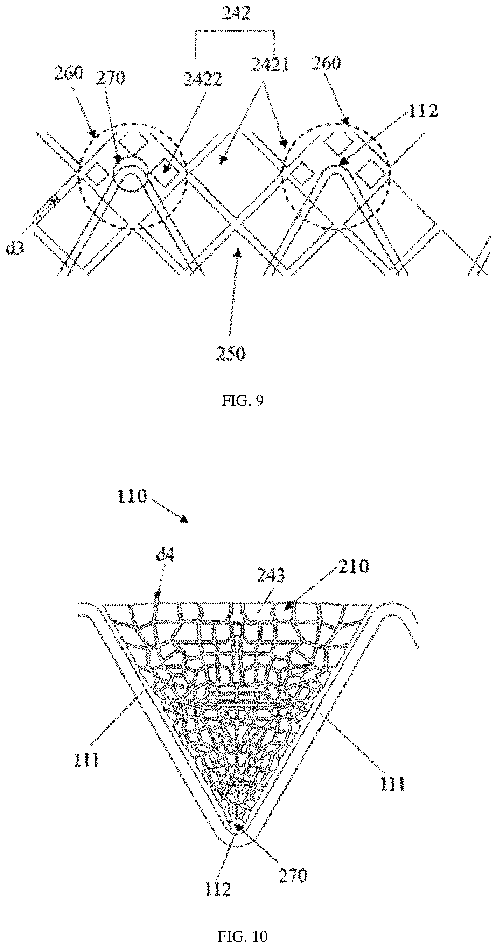

In some embodiments, the through hole 240 includes at least one of a circular hole 241 shown in FIG. 1 to FIG. 8, a diamond-shaped hole 242 shown in FIG. 9, and an amorphous hole 243 shown in FIG. 10. The diamond-shaped hole 242 may be a parallelogram, a rectangular hole, or a square hole as shown in FIG. 9. The amorphous hole 243 refers to a hole with an irregular shape, such as an arrow-shaped hole or a horn-shaped hole. Considering the difficulty of manufacturing, the through hole 240 may be formed before the membrane 200 wraps the metal stent 100. During manufacturing, the through holes 240 are formed on the membrane 200 by means of laser.

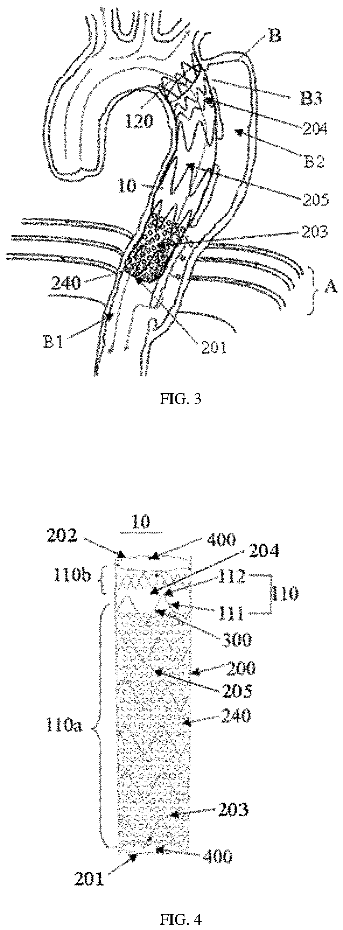

The stent graft 10 may be applied in a field of treatment of descending aortic dissecting aneurysm. The material of metallic stent 100 may be a biocompatible metal material such as nickel-titanium alloy, cobalt-nickel alloy, and stainless steel, etc. As shown in FIG. 2 and FIG. 4, the metal stent 100 may include a plurality of annular stent segments 110 spaced apart along an axial direction of the stent graft 10. Each stent segment 110 has a plurality of edges 111 and a plurality of vertices 112. The stent segment 110 is sequentially divided into a small band 110b and a large band 110a in a direction from a proximal end to a distal end of the stent graft 10. The small band 110b is mainly configured to seal the stent graft 10, each small band 110b can be of an equal-height structure or a variable-height structure, and may have 10 to 16 vertices 112. The large band 110A is mainly configured to support a blood vessel, and may have 5 to 8 vertices 112. Optionally, two stent segments 110 may be provided at the proximal end, which are arranged crosswise on the inner and outer sides of the membrane 200 (see FIG. 4), so as to improve a sealing effect of the stent graft 10.

The material of the membrane 200 may be a polymer, such as PET (Polyethylene terephthalate, polyester resin), PTFE (Poly tetra fluoroethylene, polytetrafluoroethylene), ePTFE (Expanded Polytetrafluoroethylene, expanded polytetrafluoroethylene). The membrane 200 may be fixed on the metal stent 100 by a suture, a material of the suture may be a polymer material such as PET, PTFE, ePTFE, or PP (Polypropylene).

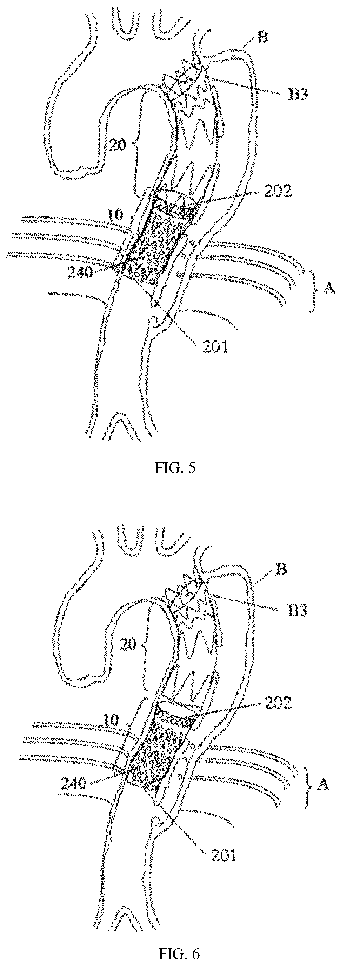

The membrane 200 includes a first membrane area 203 adjacent to the distal end 201, a second membrane area 204 adjacent to the proximal end 202, and a third membrane area 205 located between the first membrane area 203 and the second membrane area 204. The hole area 210 may be located only in the first membrane area 203 (see FIG. 2), or may be located in the first membrane area 203 and the third membrane area 205 (see FIG. 4), or may be located in the first membrane area 203, the second membrane area 204 and the third membrane area 205. As shown in FIG. 3, when the stent graft 10 is used to repair the dissection rupture B3 in a B area of the descending aorta, the hole area 210 can be located only in the first membrane area 203 to ensure a smooth blood flow in an intercostal artery A. The second membrane area 204 and third membrane area 205 were used to block a true lumen B1 and a false lumen B2 of the descending aorta B, and prevent blood from flowing into the false lumen B2 of the descending aorta B. In this case, the proximal end of the stent graft 10 may be provided with a bare stent segment 120 for anchoring the stent graft 10 in the anchoring area of the blood vessel. The bare stent segment 120 can be provided with 4 to 8 vertices 112, which can not only ensure a radial support force of the stent graft 10, but also ensure an anchoring ability of the stent graft 10 to prevent displacement. As shown in FIG. 5 and FIG. 6, the stent graft 10 can be connected to an end of a conventional main stent graft 20 to extend the main stent graft 20. The hole area 210 may be located on the first membrane area 203 and the third membrane area 205, or may be located in the first membrane area 203, the second membrane area 204, and the third membrane area 205. In this case, the stent graft 10 can ensure the smooth blood flow in the branch blood vessel while remodeling the diseased blood vessel. The stent graft 10 may overlap the conventional main stent graft 20 (see FIG. 5) or does not overlap (see FIG. 6) the conventional main stent graft 20.

According to the stent graft 10, the membrane 200 includes a hole area 210 disposed adjacent to the distal end 201, and the through holes 240 on the hole area 210 can enable the blood flow to flow from the inside of the stent graft 10 to the outside thereof. The blood in the true lumen B1 of the descending aorta B can smoothly flow into the distal intercostal artery A through the through hole 240 to ensure that the distal intercostal artery A always has blood flow and a long-term patency of the blood flow. Therefore, the problems of poor long-term fatigue performance and insufficient connection strength of the bare metal stent of the convectional stent graft 30 can be solved. The through holes 240 are formed on the membrane 200 instead of the bare metal stent segment, which can also enhance a tensile strength of the stent graft 10, ensure a flexibility of the stent graft 10 and the stability of the stent graft 10, so that the support ability of the stent graft 10 to the blood vessel can achieve an effect of remodeling the true lumen B1. In addition, by providing the through hole 240 with at least one of the circular holes 241, the diamond-shaped hole 242, and the amorphous hole 243, in practical application, the shape of the through hole 240 with the best structure can be selected according to specific conditions, which not only ensures that blood flow can flow smoothly from the inside of the stent graft 10 to the outside thereof, but also reduces the difficulty of preparing the stent graft.

In some embodiments, as shown in FIG. 2 and FIG. 4, the stent graft 10 further includes a first marker member 300 provided on the membrane 200. The first marker member 300 is located at a starting end of the hole area 210 adjacent to the distal end 201 and/or an ending end of hole area 210 adjacent to the proximal end 202. The first marker member 300 is configured to display a starting position or an ending position of the hole area 210, which is beneficial to align the hole area 210 with the blood vessel in the distal intercostal artery A, thereby ensuring smooth blood flow in the distal intercostal artery A. The first marker member 300 may be made of tantalum, platinum-iridium alloy or gold. The first marker member 300 may be fixed to the membrane 200 by sutures. The one or more first marker members 300 may be provided, for example, the two first marker members 300 are evenly arranged along a circumferential direction of the stent graft 10.

Further, the stent graft 10 further includes a second marker member 400. The second marker member 400 is located on an edge of the membrane 200 adjacent to the proximal end 202 and/or the distal end 201. The second marker member 400 is configured to display a starting position or an ending position of the stent graft 10, so that the stent graft 10 can be positioned more precisely. A material, a shape and a connection manner of the second marker member 400 can be the same as those of the first marker member 300. The one or more second marker members 400 may be provided, for example, four second marker members 400 are evenly arranged along the circumferential direction of the stent graft 10.

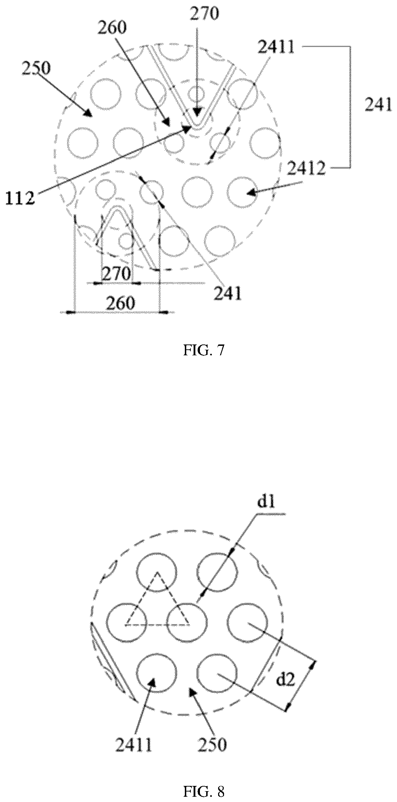

In some embodiments, as shown in FIGS. 7 and 9, the hole area 210 includes a first area 250 and a second area 260. The first area 250 is adjacent to the second area 260, the second area 260 covers the vertex 112 of the metal stent 100. An area of the through hole 240 in the first area 250 is greater than that of the through hole 240 in the second area 260. In this way, on the one hand, a connection strength between the membrane 200 and the vertex 112 of the metal stent 100 can be ensured, and the vertex 112 of the metal stent 100 can be prevented from being exposed to touch a blood vessel wall because the area of the through hole adjacent to the vertex 112 of the metal stent 100 is too large. On the other hand, the through hole away from the vertex 112 of the metal stent 100 can have a sufficient area, so that the blood can flow smoothly from the inside of the stent graft 10 to the outside thereof.

Parameters of the through holes 240 in the first area 250 and the second area 260 are described below.

In some embodiments, as shown in FIG. 7 and FIG. 8, the second area 260 refers to an area in the hole area 210 with the vertex 112 of the corresponding stent segment 110 as a center of the circle and a radius of 5mm to 7mm (e.g., 5mm, 6mm, 7mm, etc.). It should be understood that the hole area 210 of the cover membrane 200 may include a plurality of second areas 260. A number of the second areas 260 is related to a number of vertices 112 of the metal stent 100 covered by the hole area 210, the area in the hole area 210 other than the second area 260 can be regarded as the first area 250, a size of the first area 250 can be determined according to specific situations.

The through hole 240 whose circle center falls within the first area 250 is a first circular hole 2411. A diameter d1 of the first circular hole 2411 is in a range of 1mm to 6mm (e.g., 1mm, 2mm, 1mm, 3mm, 4mm, 5mm, 6mm, etc.). A distance d2 between centers of the adjacent two first circular holes 2411 is in a range of 4mm to 6mm (for example, 4mm, 4.5mm, 5mm, 5.5mm, 4mm, 6mm, etc.), the centers of the adjacent three first circular holes 2411 form an equilateral triangle (see FIG. 8).

By setting the above parameters, it can be ensured that the diameter of the first circular hole 2411 is greater than a size of the distal intercostal artery A (for example, between the 8th to the 11th intercostal), and a space between two first circular holes 2411 is also less than the size of the distal intercostal artery A (for example, between the 8th to the 11th intercostal), so as to ensure that the intercostal artery A can randomly land on the hole area 210 of the membrane 200, and to ensure that the blood in the intercostal artery A remains unobstructed. The above parameters can be set according to specific conditions. For example, the diameter d1 of the first circular hole 2411 is 2mm, the distance d2 between the centers of adjacent two first circular holes 2411 is 4mm.

Further, as shown in FIG. 7, the circular hole 241 whose circle center falls within the second area 260 is a second circular hole 2412. A diameter of the second circular hole 2412 is less than the diameter d1 of the first circular hole 2411 by 40% to 70% (for example, 40%, 50%, 60%, 70%, etc.). In some embodiments, the diameter of the second circular hole 2412 is equal to or greater than 1mm, specifically 1mm to 2mm, (e.g., 1mm, 1.2mm, 1.5mm, 2mm, etc.). By setting the above parameters, it can be ensured that the vertex 112 of the metal stent 100 will not be exposed from the second circular hole 2412, so as to avoid the vertex 112 of the metal stent 100 touching the blood vessel wall and causing secondary damage to the blood vessel wall.

Further, in some embodiments, a distance between any point on an edge of the second circular hole 2412 and the vertex 112 of the corresponding stent segment 110 in the second area 260 is greater than or equal to 3cm (For example, 3cm, 3.2cm, 3.5cm, etc.). That is, in the second area 260, no circular hole 241 is provided in a third area 270 with the vertex 112 of the stent segment 110 as the center and the radius of 3mm. In this way, it can be ensured that the vertex 112 of the metal stent 100 can be firmly fixed on the membrane 200.

It should be noted that when the diameter d1 of the first circular hole 2411 is 6mm and the diameter of the second circular hole 2412 is 40% less than the diameter d1 of the first circular hole 2411, the diameter of the second circular hole 2412 can be is 3.6mm, and the distance between the second circular hole 2412 and the vertex 112 of the corresponding stent segment 110 in the second area 260 is greater than or equal to 3cm, so the second circular hole 2412 can cover the vertex 112.

In some embodiments, as shown in FIG. 9, the membrane 200 includes the first area 250 and the second area 260. The size ranges of the first area 250 and the second area 260 are the same as that of the embodiment shown in FIG. 7. The through hole 240 whose center falls within the first area 250 is a first diamond-shaped hole 2421. A plurality of the first diamond-shaped holes 2421 are arranged in a matrix. An area of each first diamond-shaped hole 2421 is in a range of 0.78mm² to 7mm² (e.g., 0.78mm², 1mm², 3mm², 5mm², 7mm², etc.). An edge distance d3 between the adjacent two first diamond-shaped holes 2421 is in a range of 1mm to 3mm (for example, 1cm, 2cm, 3cm, etc.), so that the diameter of the intercostal artery is greater than the edge distance of the adjacent first diamond-shaped holes 2421, so that the intercostal artery blood vessels can be unobstructed. It should be noted that the edge distance d3 of the adjacent first diamond-shaped holes 2421 refers to the shortest distance between the sides of adjacent two first diamond-shaped holes 2421. In addition, a center line connecting the adjacent two first diamond-shaped holes 2421 in an axial direction of the stent graft 10 is parallel to an axis of the stent graft 10. The centers of the plurality of first diamond-shaped holes 2421 in the circumferential direction of the stent graft 10 are coplanar so that the first diamond-shaped holes 2421 are arranged in a matrix. By setting the above parameters, it can be ensured that the area of the first diamond-shaped hole 2421 is greater than a cross-sectional area of the distal intercostal artery A (e.g., between the 8th to the 11th intercostal), and a distance between the edges of the adjacent two first diamond-shaped holes 2421 is less than the cross-sectional area of the distal intercostal artery A (for example, between the 8th to the 11th intercostal), which also enables the intercostal artery A to randomly land on the hole area 210, thereby ensuring that the blood in the intercostal artery A remains unobstructed. The size of the first area 250 can be set according to specific conditions. Taking the first diamond-shaped hole 2421 in the first membrane area 203 as an example, the first area 250 may refer to the entire first membrane area 203, or may refer to the area of the first membrane area 203 adjacent to the blood vessel of the intercostal artery A.

Further, in some embodiments, as shown in FIG. 9, the through hole 240 whose center falls within the second area 260 is a second diamond-shaped hole 2422. An area of the second diamond-shaped hole 2422 is less than that of the first diamond-shaped hole 2421 by 40% to 70% (e.g., 40%, 50%, 60%, 70%, etc.). By setting the above parameters, it can be ensured that the vertex 112 of the metal stent 100 will not be exposed from the second diamond-shaped hole 2422, so as to avoid the vertex 112 of the metal stent 100 touching the blood vessel wall and causing secondary damage to the blood vessel wall.

Further, in some embodiments, a distance between any point on an edge of the second diamond-shaped hole 2422 and the vertex 112 of the corresponding stent segment 110 is greater than or equal to 3cm (e.g., 3cm, 3.2cm, 3.5cm, etc.). That is, no diamond-shaped hole 242 is provided in the third area 270 with the vertex 112 of the corresponding stent segment 110 as the center and the radius of 3mm. In this way, it can be ensured that the vertex 112 of the metal stent 100 can be firmly fixed on the membrane 200.

As shown in FIG. 10, in some embodiments, the metal stent 100 includes the plurality of stent segments 110 spaced apart in the axial direction thereof. Each stent segment 110 has a plurality of edges 111 and a plurality of vertices 112, the hole area 210 is formed between adjacent two edges of each stent segment 110. The through hole 240 in the hole area 210 is the amorphous hole 243. The amorphous hole 243 is arranged to avoid the stent segments 110 of the metal stent 100. An area of the amorphous hole 243 is in a range of 0.78mm² to 7mm²(e.g., 0.78mm², 1mm², 3mm², 5mm², 7mm², etc.), an edge distance d4 of adjacent two amorphous holes 243 is in a range of 1mm to 3mm (e.g., 1mm, 1.5mm, 2mm, 2.5mm, 3mm, etc.). It should be noted that the edge distance d4 of the amorphous holes 243 refers to the shortest distance between the sides of adjacent two amorphous holes 243. The setting of the above parameters can not only ensure the strength of the membrane 200, but also ensure that the diameter of the intercostal artery A is greater than the edge distance between adjacent two amorphous holes 243, so that the blood vessels of the intercostal artery A can be unobstructed.

Further, in some embodiments, as shown in FIG. 10, the areas of the amorphous holes 243 gradually increase along a direction away from the vertex 112 between the adjacent two edges 111. In this way, the strength of the membrane 200 located adjacent to the vertex 112 of the metal stent 100 can be ensured, and smooth blood flow can be obtained.

As shown in FIG. 10, further, the distance between any point on the edge of the amorphous hole 243 and the vertex 112 of the corresponding stent segment 110 is greater than or equal to 3cm (for example, 3cm, 3.2cm, 3.5cm, etc.). That is, no amorphous hole 243 is provided in the third area 270 with the vertex 112 of the corresponding stent segment 110 as the center and the radius of 3mm. In this way, it can be ensured that the vertex 112 of the metal stent 100 can be firmly fixed on the membrane 200.

In some embodiments, the through holes 240 are formed on the membrane 200 by means of laser drilling. The method of forming the hole area 210 is simple, which is convenient for the manufacturing of the stent graft 10. During production, the plurality of hole areas 210 may be formed on the distal end of the membrane 200 before the membrane 200 wraps the metal stent 100, so that a difficulty of forming holes on the membrane 200 can be reduced.

According to another embodiment, a stent graft system is also provided. As shown in FIG. 5 and FIG. 6, the stent graft system includes at least one main stent graft 20 and the stent graft 10 described in any of the above embodiments. The main stent graft 20 and the stent graft 10 are sequentially arranged along a blood flow direction.

A structure and a material of the main stent graft 20 may be the same as a structure and a material of the conventional stent graft, which will not be repeated here. The number of main stent graft 20 may be set according to specific conditions. For example, as shown in FIG. 5 and FIG. 6, one main stent graft 20 is provided.

In the above-mentioned stent graft system, the membrane 200 of the stent graft 10 includes at least the plurality of hole areas 210 provided adjacent to the distal end 201, the hole areas 210 can allow the blood to flow from the inside of the stent graft 10 to the outside thereof, the blood in the true lumen B1 of the descending aorta B can smoothly flow into the distal intercostal artery A through the hole area 210 of the membrane 200, so as to ensure that the distal intercostal artery A always has the blood flow and the long-term patency of the blood flow. Therefore, the problems of poor long-term fatigue performance and insufficient connection strength of the bare metal stent of the traditional stent graft can be solved. In addition, the hole area 210 is disposed on the membrane 200 instead of the bare metal stent segment, which can also enhance the tensile strength of the stent graft 10, ensure the flexibility of the stent graft 10 and the stability of the stent graft 10, so that the support ability of the stent graft 10 to the blood vessel can achieve an effect of remodeling the true lumen B1.

The proximal end of the stent graft 10 overlaps (see FIG. 5) or does not overlap (see FIG. 6) a distal end of the adjacent main stent graft 20. When in use, it can be determined according to the specific situation.

Although the respective embodiments have been described one by one, it shall be appreciated that the respective embodiments will not be isolated. Those skilled in the art can apparently appreciate upon reading the disclosure of this application that the respective technical features involved in the respective embodiments can be combined arbitrarily between the respective embodiments as long as they have no collision with each other. Of course, the respective technical features mentioned in the same embodiment can also be combined arbitrarily as long as they have no collision with each other.

The foregoing descriptions are merely specific embodiments of the present disclosure, but are not intended to limit the protection scope of the present disclosure. Any variation or replacement readily figured out by a person skilled in the art within the technical scope disclosed in the present disclosure shall all fall within the protection scope of the present disclosure. Therefore, the protection scope of the present disclosure shall be subject to the protection scope of the appended claims.

## Claims

1. A stent graft comprising:
a metal stent; and
a membrane wrapping the metal stent and comprising a proximal end and a distal end, wherein the membrane comprises a hole area adjacent to the distal end, the hole area is provided with a plurality of through holes configured to allow blood to flow from an inside of the metal stent to an outside thereof.

2. The stent graft according to claim 1, wherein the metal stent comprises a plurality of stent segments spaced apart in an axial direction thereof, each stent segment has a plurality of edges and a plurality of vertices, and the hole area comprises a first area and a second area, the first area is adjacent to the second area, the second area covers one of the vertices, an area of the through hole in the first area is greater than an area of the through hole in the second area.

3. The stent graft according to claim 2, wherein the through hole in the first area is a first circular hole, a diameter of the first circular hole is in a range of 1mm to 6mm, a distance between centers of adjacent two first circular holes is in a range of 4mm to 6mm, the centers of adjacent three first circular holes form an equilateral triangle.

4. The stent graft according to claim 3, wherein the second area comprises an area with the vertex as a center and a radius of 5mm to 7mm, the through hole in the second area is a second circular hole, a diameter of the second circular hole is 40% to 70% less than the diameter of the first circular hole.

5. The stent graft according to claim 4, wherein a distance between any point on an edge of the second circular hole and the vertex is greater than or equal to 3cm.

6. The stent graft according to claim 2, wherein the through hole in the first area is a first diamond-shaped hole, an area of the first diamond-shaped hole is in a range of 0.78mm² to 7mm², an edge distance between adjacent two first through holes is in a range of 1mm to 3mm, the first diamond-shaped holes are arranged in a matrix.

7. The stent graft according to claim 6, wherein the second area comprises an area with the vertex as a center and a radius of 5mm to 7mm, the through hole in the second area is a second diamond-shaped hole, an area of the second diamond-shaped hole is 40% to 70% less than the area of the first diamond-shaped hole.

8. The stent graft according to claim 7, wherein a distance between any point on an edge of the second diamond-shaped hole and the vertex is greater than or equal to 3cm.

9. The stent graft according to claim 1, wherein the metal stent comprises a plurality of stent segments spaced apart in an axial direction thereof, each stent segment has a plurality of edges and a plurality of vertices, the hole area is located between adjacent two edges of each stent segment, the through hole in the hole area is an amorphous hole, an area of the amorphous hole is in a range of 0.78mm² to 7mm², an edge distance between adjacent two amorphous holes is in a range of 1mm to 3mm.

10. The stent graft according to claim 9, wherein a distance between any point on an edge of the amorphous hole and the vertex is greater than or equal to 3cm.

11. The stent graft according to claim 9, wherein the areas of the amorphous holes gradually increase along a direction away from the vertex between the adjacent two edges.

12. The stent graft according to claims 1, further comprising a first marker member provided on the membrane, wherein the first marker member is located at a starting end of the hole area adjacent to the distal end and/or an ending end of hole area adjacent to the proximal end.

13. The stent graft according to claims 1, further comprising a second marker member provided on the membrane, wherein the second marker member is located on an edge of the membrane adjacent to the proximal end and/or the distal end.

14. A stent graft system comprising:
a main stent graft; and
a stent graft according to any one of claims 1 to 13, wherein the main stent graft and the stent graft are sequentially arranged along a blood flow direction.
